# EUROPÄISCHE PATENTSCHRIFT

(11) **EP 2 283 899 B1**
(45) Veröffentlichungstag und Bekanntmachung des Hinweises auf die Patenterteilung: **19.07.2017**
(21) Anmeldenummer: 10177131.9
(22) Anmeldetag: 19.03.2007
(51) Int. Cl.: A61Q 5/08, A61K 8/46, A61K 8/49, D21H 21/32, D06L 4/30

(54) **REDUKTIVER FARBABZUG**
REDUCTIVE COLOUR REMOVAL
DÉCOLORATION RÉDUCTIVE

(30) Priorität: 21.03.2006 DE 102006013260; 11.05.2006 DE 102006022215
(43) Veröffentlichungstag der Anmeldung: 16.02.2011
(62) Teilanmeldung aus: 07723365.8
(73) Patentinhaber: Henkel AG & Co. KGaA, 40589 Düsseldorf (DE)
(72) Erfinder: Müller, Burkhard, 21075 Hamburg (DE); Neubueser, Inge, 22587 Hamburg (DE); Groß, Wibke, 41836 Hückelhoven (DE)

(56) Entgegenhaltungen:
- WO-A1-99/18067
- WO-A1-03/026597
- BE-A- 648 012
- DE-B- 1 081 859
- FR-A1- 2 814 948
- FR-A1- 2 829 764

## Beschreibung

Die Erfindung betrifft Zusammensetzungen zum reduktiven Farbabzug, welche organische Sulfinsäurederivate sowie zusätzliche Verbindungen, ausgewählt aus den Aldehyden und/oder den Ketonen, enthält und einen Gehalt an Sulfitanionen von höchstens 1,5 Gew.-% besitzt. Diese Farbabzugsmittel eignen sich für die verbesserte Entfärbung gefärbter Substrate wie Papier, Textilien und insbesondere keratinhaltigen Fasern, beispielsweise menschlicher Haare. Gleichfalls ist ein Verfahren zur Entfärbung gefärbter Substrate, sowie die Verwendung besagter Mittel zum Farbabzug Gegenstand der Erfindung.

Beim Färben wird der Farbstoff auf das Substrat durch Adsorption an die Oberfläche, durch Eindiffundieren, durch Bildung auf und/oder in dem Substrat bzw. durch chemische Bindung übertragen. Für das Färben, beispielsweise von Papier, Textilien oder keratinhaltigen Fasern, kommen im allgemeinen entweder direktziehende Farbstoffe oder Oxidationsfarbstoffe in Frage. Oxidationsfarbstoffe entstehen durch oxidative Kupplung einer oder mehrerer Entwicklerkomponenten untereinander oder mit einer oder mehreren Kupplerkomponenten. Kuppler- und Entwicklerkomponenten werden auch als Oxidationsfarbstoffvorprodukte bezeichnet. Die oxidative Kupplung findet bevorzugt während der Färbevorgangs statt, damit die Farbstoffvorprodukte in das Substrat hinein diffundieren können und der Farbstoff sich in dem Substrat bildet. Durch die Größe des entstandenen Farbstoffmoleküls wird ein Auswaschen aus dem Substrat erschwert.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.
Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Unter direktziehenden Farbstoffen werden im allgemeinen Farbstoffe verstanden, die bereits vor Beginn des Färbens vorgebildet sind und auf das Substrat aufziehen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Ein wichtiges technisches Gebiet stellt neben dem Färben das Abziehen von Farbstoffen dar. Darunter versteht man im Allgemeinen das Entfernen von Färbungen oder Bedruckungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung. Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Textilien oder Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.
Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch Färbung aufgetragenen Farbstoffe reduktiv ein. Es tritt somit kaum eine Aufhellung des Haars auf. Allerdings bedürfen die reduktiven Entfärbemittel einer Verbesserung der Entfärbeleistung.
Überwiegend nutzt man reduktiv wirkende Schwefelverbindungen zur Entfärbung. Bekanntermaßen eignen sich Dithionite oder Derivate der 1-Hydroxymethylsulfinsäure bzw. der 1-Aminomethylsulfinsäure als Reduktionsmittel.

Aus der Druckschrift US-A-3 892 845 sind dem Fachmann reduktive Farbabzugsmittel bekannt, mit deren Hilfe sich gefärbte keratinhaltige Fasern entfärben lassen. Als Reduktionsmittel ist 1-Hydroxymethylsulfinsäure oder ein Salz davon in den Entfärbemitteln enthalten. Gemäß DE-OS-1 617 829 lässt sich durch Zugabe von 1-Hydroxymethylsulfinsäure zu oxidativen Entfärbemitteln, enthaltend Wasserstoffperoxid und Persulfate, deren Bleichwirkung verstärken.
Laut Offenbarung der EP-A-1 079 018 eignen sich Aminoalkansulfinate der Formel R¹_{3-z}N (CR²R³-SO₂-M)_{z} (R², R³ = Wasserstoff oder (C₁ bis C₄)-Alkyl) zur teilweisen Entfärbung von mit Küpenfarbstoffen oder Schwefelfarbstoffen gefärbten oder bedruckten Textilien.
Gemäß WO-A1-99/18067 eignen sich 1-Hydroxyalkylsulfinsäuren bzw. 1-Aminoalkylsulfinsäuren, welche eine Carboxygruppe, eine Sulfonsäuregruppe, eine Acylgruppe, eine Aminocarbonylgruppe oder eine Alkoxycarbonylgruppe tragen, zur Entfärbung gefärbter Substrate. Besagte Derivate besitzen eine vergleichbare oder bessere Entfärbekraft wie die 1-Hydroxymethylsulfinsäure (*vide supra*). In der WO-A1-02/30369 wird die Entdeckung beschrieben, dass sich mit den Sulfinsäure-Derivaten der WO-A1-99/18067 auch keratinhaltige Fasern, wie beispielsweise Haare, entfärben lassen. Weitere zur Entfärbung keratinhaltiger Fasern geeignete Sulfinsäurederivate finden sich in den Druckschriften WO-A1-03/026597 und WO-A1-03/41668.

Substrate, die mit den Sulfinsäurederivaten des Standes der Technik reduktiv entfärbt wurden, erfahren einige Zeit nach dem Farbabzug eine unerwünschte Nachdunkelung. Ferner enthalten die beschriebenen Entfärbemittel überwiegend Sulfitanionen.

Aufgabe der vorliegenden Erfindung ist es, in der Entfärbeleistung verbesserte reduktive Entfärbemittel bereitzustellen, die das Substrat dauerhaft ohne Nachdunkelung entfärben. Die Substratstruktur soll dabei geschont werden. Ferner sollten die in den Entfärbemitteln eingesetzten Reduktionsmittel für eine kosmetische Verwendung physiologisch verträglich und toxikologisch unbedenklich sein.
Überraschenderweise wird die Aufgabe durch Mittel gelöst, die mindestens eine der erfindungsgemäßen organischen Sulfinsäurederivate sowie zusätzlich Verbindungen, ausgewählt aus den Aldehyden und/oder den Ketonen, enthalten und einen Maximalgehalt von Sulfitanionen nicht überschreitet. Die Entfärbung gefärbter Substrate, wie beispielsweise Papier, Textilien oder keratinhaltige Fasern, insbesondere menschliches Haar, gelingt verbessert ohne dass sich nach dem Entfärbevorgang eine Nachdunkelung in dem hohen Maße einstellt, wie sie durch die Verwendung der Sulfinsäurederivate allein hervorgerufen wird. Die erfindungsgemäßen Mittel eignen sich besonders zur faserschonenden Entfärbung keratinhaltiger Fasern.

Unter keratinhaltigen Fasern sind beispielsweise Wolle, Pelze, Federn und insbesondere menschliche Haare zu verstehen.

Ein erster Gegenstand der Erfindung sind daher Mittel, die in einem Träger mindestens ein Sulfinsäurederivat der Formel (la-8) enthalten 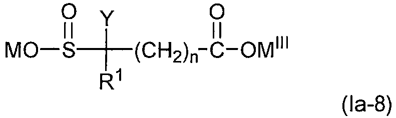 worin
- M: steht für ein Wasserstoffatom oder ein Äquivalent eines ein- oder mehrwertigen Kations,
- Y: für eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe stehen,
- M'": steht unabhängig von M für die unter M aufgeführten Merkmale,
- R¹: für ein Wasserstoffatom oder eine Methylgruppe steht,
wobei höchstens 1,5 Gew.-% an Sulfitanionen, bezogen auf das Gewicht des anwendungsbereiten Mittels, enthalten sind und
wobei es zusätzlich Verbindungen, ausgewählt aus den Aldehyden und/oder den Ketonen, enthält.

Falls die Verbindungen gemäß Formel (Ia-8) mindestens ein Chiralitätszentum enthalten, sind selbstverständlich alle Stereoisomere allein sowie deren Mischungen, insbesondere deren Racemate, erfindungsgemäß.

Die Verbindungen gemäß Formel (la-8) können neben der Form als freie Säure oder als dessen Salz auch als inneres Salz vorliegen.

Wenn die Verbindung der Formel (la-8) als Säure vorliegt, bedeuten die Reste M-und/oder M^{III} ein Wasserstoffatom. Die Fragmente MO- und M^{III}O- der Formel (Ia-8) bilden in diesem Fall eine Hydroxygruppe. Wenn die Sulfinsäure der Formel (la-8) als Salz vorliegt, steht mindestens einer der Reste M oder M^{III} für ein Äquivalent eines ein- oder mehrwertigen Kations. Das ein oder mehrwertige Kation M^{z+} mit einer Ladungszahl z von eins oder höher dient lediglich aus Gründen der Elektroneutralität zur Kompensation der einfach negativen Ladung des bei Salzbildung 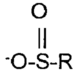 vorliegenden Sulfinat-Fragments bzw. des Carboxylat-Fragments der Formel (Ia-8). Das dafür zu verwendende Äquivalent des entsprechenden Kations beträgt 1/z. Das Fragment MO- der Formel (la-8) steht im Fall der Salzbildung für die Gruppe: 1/z (M^{z+}) -O-. Gleiches gilt mutatis mutandis für M^{III}.

Als entsprechende ein- oder mehrwertige Kationen kommen prinzipiell alle Kationen in Frage, die keine Redox-Reaktion mit dem übrigen Sulfinat-Fragment der Formel (la-8) eingehen. Insbesondere sind dies Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (la-8) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M, M', M" und M^{III} stehen bevorzugt unabhängig voneinander für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Das Äquivalent des gegebenenfalls vorhandenen ein- oder mehrwertigen Anions gemäß Formel (Ia-8) wird, analog zur Definition der Kationäquivalente, zur Wahrung der Elektroneutralität durch Formulierung eines stöchiometrischen Koeffizienten kleiner 1 vor der Bezeichnung des Anions beschrieben. Die besagten Anionen werden im Weiteren definitionsgemäß mit An⁻ symbolisiert. Sie werden bevorzugt ausgewählt aus Halogenid, ½ Sulfat, Hydrogensulfat, ½ Carbonat, Hydrogencarbonat, 1/3 Phosphat, ½ Hydrogenphosphat, Dihydrogenphosphat oder p-Toluolsulfonat. Besonders bevorzugt steht das Anion für Chlorid, Bromid, p-Toluolsulfonat oder Hydrogensulfat.

Es sind solche Verbindungen der Formel (la-8) erfindungsgemäß bevorzugt, bei welchen Y eine Hydroxy- oder Aminogruppe bedeutet.

Bevorzugt steht n in Formel (la-8) für eine Zahl 0, 1 oder 2.

Im Folgenden sollen Beispiele für die als Substituenten im Rahmen dieser Anmeldung genannten Gruppen bzw. Reste erwähnt werden. Beispiele für (C₁ bis C₆)-Alkylreste sind lineare, verzweigte oder zyklische (C₁ bis C₆)-Alkylgruppen, wobei lieneare oder verzweigte (C₁ bis C₆)-Alkylgruppen bevorzugt sind. Insbesondere sind die Gruppen Methyl, Ethyl, Propyl, Isopropyl, n-Butyl, sec-Butyl, tert.-Butyl, n-Pentyl und n-Hexyl geeignet. Beispiele für entsprechend geeignete zyklische Alkylgruppen sind Cyclopentyl und Cyclohexyl.
Beispiele für bevorzugte (C₂ bis C₆)-Alkenylreste sind Vinyl, Allyl und Butenyl. Weiterhin können als bevorzugte Beispiele für eine (C₁ bis C₆)-Monohydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe genannt werden. Eine 2-Hydroxyethylgruppe ist besonders bevorzugt.
Beispiele für eine (C₂ bis C₆)-Polyhydroxyalkylgruppe sind die 2,3-Dihydroxypropylgruppe, 3,4-Dihydroxybutylgruppe und die 2,4-Dihydroxybutylgruppe. Bevorzugte Arylgruppen sind Phenyl, Naphthyl und Biphenyl.
Bevorzugte Aryl-(C₁ bis C₆)-alkylgruppen sind Benzyl und 2-Phenylethyl.

Die weiteren verwendeten Begriffe leiten sich erfindungsgemäß von den hier gegebenen Definitionen ab.

Ganz besonders bevorzugte Vertreter der Sulfinsäurederivate gemäß Formel (la-8) sind die Sulfinsäuren gemäß folgender Liste oder deren Salze mit einem Äquivalent mindestens eines ein- oder mehrwertigen Kations:

| Name: | korrespondierende Struktur: |
|---|---|
| 4-Hydroxy-4-sulfinobutansäure, | 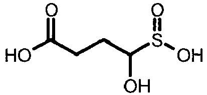 |
| 4-Hydroxy-4-sulfinopentansäure, | 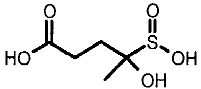 |
| 5-Hydroxy-5-sulfinopentansäure, | 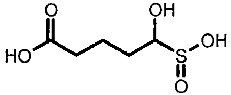 |
| 5-Hydroxy-5-sulfinohexansäure, | 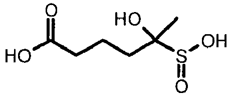 |
| 2-Hydroxy-2-sulfino-essigsäure | 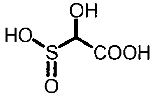 |
| 2-Amino-2-sulfino-essigsäure | 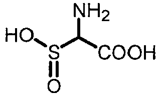 |
| 2-Hydroxy-2-sulfino-propionsäure | 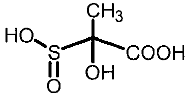 |
| 2-Amino-2-sulfino-propionsäure | 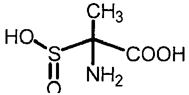 |
| 3-Hydroxy-3-sulfinato-propionsäure | 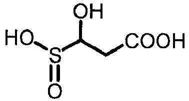 |
| 3-Amino-3-sulfinato-propionsäure | 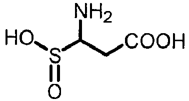 |

Für die bevorzugten ein- oder mehrwertigen Kationen aller Salze der zuvor genannten Verbindungen gilt das zuvor Gesagte.

Die Herstellung der erfindungsgemäß eingesetzten Sulfinsäuren der Formel (la-8) erfolgt beispielsweise aus der Umsetzung von Dithionit mit entsprechenden Aldehyden bzw. Ketonen analog zu der Herstellvorschrift gemäß WO-A1-99/18067.

Bevorzugt enthält das erfindungsgemäße Mittel mindestens ein Sulfinsäurederivat der Formel (la-8) in einer Menge von 0,01 bis 20 Gew.-%, besonders bevorzugt von 1 bis 20 Gew.-%, jeweils bezogen auf das Gewicht des Mittels.

Wenn das Sulfitanion lediglich in geringer Menge zugesetzt bzw. gar nicht enthalten ist fällt überraschenderweise die Schädigung der Substrate geringer aus, als bei entsprechenden sulfinsäurehaltigen Entfärbemitteln mit hohem Sulfitgehalt.

Die Menge an Sulfitanion (SO₃²⁻, Molmasse 80,06 g/mol) berechnet sich erfindungsgemäß als Gewichtsprozent an Sulfitanion der enthaltenen sulfithaltigen Verbindungen, das heißt Gegenionen und andere Komponenten der sulfithaltigen Verbindungen werden bei der Berechnung nicht berücksichtigt. Es ist erfindungsgemäß bevorzugt, wenn die erfindungsgemäßen Mittel höchstens 1,5 Gew.-%, besonders bevorzugt höchstens 1,0 Gew.-%, ganz besonders bevorzugt höchstens 0,5 Gew.-%, an Sulfitanionen, jeweils bezogen auf das Gewicht des anwendungsbereiten Mittels, enthalten.

Es werden in einer bevorzugten Ausführungsform solche Mittel eingesetzt, die in einem Träger mindestens eine Sulfinsäure der obenstehenden Formel (la-8) enthalten, mit der Maßgabe, daß alle zur Herstellung dieses Mittels eingesetzten Rohstoffe in ihren zur Bereitstellung des erfindungsgemäßen Mittels angewandten Mengen vor der Mischung in Summe höchstens 1,5 Gew.-%, bevorzugt höchstens 1,0 Gew.-%, besonders bevorzugt höchstens 0,5 Gew.-% an Sulfitanionen, jeweils bezogen auf das Gewicht des aus besagten Rohstoffen bereitgestellten, anwendungsbereiten Mittels, enthalten. Der Sulfitanionengehalt wird wie zuvor beschrieben berechnet.

Soll das anwendungsbereite Mittel bei saurem pH-Wert angewendet werden, kann das anwendungsbereite Mittel bereits als fertige Anwendungsmischung mit saurem pH-Wert angeboten werden. Es kann jedoch günstig sein das anwendungsbereite Mittel erst unmittelbar vor oder während der Anwendung am Substrat herzustellen. Dazu werden, entweder
(a) eine Zusammensetzung mit einem pH-Wert größer pH 7, enthaltend mindestens eine Sulfinsäure gemäß obiger Formel (Ia-8) bzw. deren korrespondierendes Salz und zusätzlich mindestens eine Verbindung, ausgewählt aus Aldehyden und/oder Ketonen, kurz vor oder während der Anwendung am Substrat mit einer säurehaltigen Zusammensetzung vermischt, so dass der pH-Wert auf einen pH von höchstens 7 herabgesetzt wird, oder
(b) eine wasserfreie Zusammensetzung, enthaltend mindestens eine Sulfinsäure gemäß obiger Formel (Ia-8) bzw. deren korrespondierendes Salz und zusätzlich mindestens eine Verbindung, ausgewählt aus Aldehyden und/oder Ketonen, kurz vor oder während der Anwendung am Substrat mit einer wasserhaltigen Zusammensetzung mit einem sauren pH-Wert vermischt,
jeweils mit der Maßgabe, dass
(i) das anwendungsbereite Mittel oder
(ii) alle zur Herstellung dieses Mittels eingesetzten Rohstoffe in ihren zur Bereitstellung des erfindungsgemäßen Mittels angewandten Mengen vor der Mischung in Summe
höchstens 1,5 Gew.-%, bevorzugt höchstens 1,0 Gew.-%, besonders bevorzugt höchstens 0,5 Gew.-% an Sulfitanionen, jeweils bezogen auf das Gewicht des anwendungsbereiten Mittels, enthalten.

Darüber hinaus müssen die in dem erfindungsgemäßen Mittel enthaltenen Mengen an Sulfitanion nicht zwingend vollständig in dem Träger gelöst sein. Es stellte sich heraus, dass sich auch ungelöste sulfitanionenhaltige Verbindungen nachteilig auf die Parameter der Haarsschädigung und der Stärke der Aufhellung auswirken, solange sich diese sulfitanionenhaltigen Verbindungen zumindest teilweise lösen.

Erfindungsgemäß enthalten die Mittel zusätzlich mindestens eine Verbindung, ausgewählt aus den Aldehyden und/oder den Ketonen. Der Auswahl der Verbindungen aus den Aldehyden bzw. den Ketonen ist zunächst keine Grenze gesetzt. Als besonders geeignet erweist sich mindestens ein Vertreter aus der Gruppe, die gebildet wird aus:
- Oxocarbonsäuren oder deren Salze,
- Oxocarbonsäureester,
- cyclische, lineare oder verzweigte aliphatische Aldehyde,
- cyclische, lineare oder verzweigte aliphatische Ketone,
- mono- bis polyhydroxyfunktionalisierte Aldehyde,
- mono- bis polyhydroxyfunktionalisierte Ketone,
- alicyclische, aromatische oder heterocyclische Aldehyde sowie
- alicyclische, aromatische oder heterocyclische Ketone.

Generell gilt, dass in den oben genannten erfindungsgemäßen Verbindungen der cyclischen, alicyclischen bzw. heterocyclischen Ketone die Ketogruppe(n) endocyclisch (und/oder) oder exocyclisch gebunden sein kann (können).

Oxocarbonsäuren sind organische Verbindungen, die neben mindestens einer Carboxylgruppe eine Carbonylgruppe tragen und sind somit Aldehyd- bzw. Ketocarbonsäuren. Bevorzugte Oxocarbonsäuren sind α-Oxocarbonsäuren, ß-Oxocarbonsäuren, γ-Oxocarbonsäuren sowie w-Oxocarbonsäuren der Formel (XII) bzw. deren Salze, 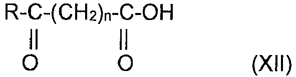 worin bedeuten
- R: ein Wasserstoffatom, eine (C₁ bis C₆)-Alkylgruppe, eine (C₁ bis C₆)-Hydroxyalkylgruppe, eine gegebenenfalls substituierte Arylgruppe, eine gegebenenfalls substituierte Heteroarylgruppe, eine (C₂ bis C₆)-Alkenylgruppe oder eine Carboxy-(C₁ bis C₆)-alkylgruppe,
- n: eine Zahl 0, 1, 2 oder 3.

Besonders bevorzugt werden die Oxocarbonsäuren ausgewählt aus mindestens einem Vertreter aus der Gruppe Glyoxalsäure, Acetessigsäure, 3-Oxoglutarsäure, 4-Oxovaleriansäure und Brenztraubensäure bzw. den Salzen der vorgenannten Säuren.

Die Oxocarbonsäureester werden bevorzugt ausgewählt aus (C₁ bis C₆)-Alkylestern der erfindungsgemäß bevorzugten Oxocarbonsäuren.

Unter cyclischen, linearen oder verzweigten aliphatischen Aldehyden sind erfindungsgemäß aliphatische Aldehyde zu verstehen, deren Formylgruppe(n) nicht in Konjugation mit einem aromatischen π-Elektronen-System stehen. Die entsprechenden Aldehyde dürfen aromatische Reste tragen, solange die π-Elektronen der Formylgruppe(n) nicht über ein solches aromatisches System delokalisiert sein können. Bevorzugte cyclische, lineare oder verzweigte aliphatische Aldehyde sind gesättigt oder ungesättigt und werden besonders bevorzugt ausgewählt aus mindestens einem Vertreter aus der Gruppe Formaldehyd, Acetaldehyd, Glyoxal, Propionaldehyd, Butanal, Pentanal, Isopentanal, Hexanal, Cyclohexanal, Heptanal, Octanal, Malondialdehyd, Glutaraldehyd, 2-Methylpentanal, 2-Ethylhexanal, 3,5,5-Trimethylhexanal, 2-Ethylbutyraldehyd, 2-Methylbutyraldehyd, Isobutyraldehyd, 3-Phenylpropanal, 3-(4-Methylphenyl)propanal, 3-(4-Methoxyphenyl)propanal, 3-(2-Methoxyphenyl)propanal, 2-Butenal, Acrolein, 3-Methyl-2-butenal, 3,7-Dimethyl-2,6-octadienal, 2,4-Pentadienal, 3,7-Dimethyl-6-octenal, 2,4-Dimethyl-3-cyclohexencarboxaldehyd und 2,6-Nonadienal.

Unter cyclischen, linearen oder verzweigten aliphatischen Ketonen sind erfindungsgemäß aliphatische Ketone zu verstehen, deren Ketogruppe(n) nicht in Konjugation mit einem aromatischen π-Elektronen-System stehen. Die entsprechenden Ketone dürfen jedoch aromatische Reste tragen, solange die π-Elektronen der Ketogruppe(n) nicht über ein solches aromatisches System delokalisiert sein können. Bevorzugte cyclische, lineare oder verzweigte aliphatische Ketone sind gesättigt oder ungesättigt und ausgewählt aus mindestens einem Vertreter aus der Gruppe Aceton, 2-Butanon, 2-Pentanon, 3-Pentanon, 2-Hexanon, 3-Hexanon, Butan-2,4-dion, Pentan-2,4-dion, Hexan-2,5-dion, Cyclohexanon, Cyclopentanon, 4-Methylpentan-2-on, 5-Methyl-3-hexen-2-on, 2-(3-Oxopropyl)benzoesäuremethylester und 4-(3-Oxopropyl)benzoesäuremethylester.

Bevorzugt verwendbare monohydroxyfunktionalisierte Aldehyde werden ausgewählt aus mindestens einem Vertreter aus der Gruppe 1-Hydroxypropanal, 5-Hydroxypentanal, 3,7-Dimethyl-7-hydroxyoctanal, Hydroxyisohexyl-3-cyclohexen-1-carboxaldehyd und 2,6,6-Trimethyl-1,3-cyclohexadien-1-carboxaldehyd.

Polyhydroxyfunktionalisierte Aldehyde sind erfindungsgemäß bevorzugt die sogenannten Aldosen und werden besonders bevorzugt ausgewählt aus mindestens einem Vertreter aus der Gruppe 2,3-Dihydroxypropionaldehyd, D-Erythrose, D-Threose, D-Ribose, D-Arabinose, D-Lyxose, D-Xylose, D-Allose, D-Altrose, D-Galactose, D-Glucose, D-Idose, D-Mannose, D-Rhamnose und D-Talose, sowie den L-Konfigurationen L-Erythrose, L-Threose, L-Ribose, L-Arabinose, L-Lyxose, L-Xylose, L-Allose, L-Altrose, L-Galactose, L-Glucose, L-Idose, L-Mannose, L-Rhamnose und L-Talose.

Monohydroxyfunktionalisierte Ketone, die sich erfindungsgemäß besonders eignen, werden bevorzugt ausgewählt aus mindestens einem Vertreter aus der Gruppe 1-Hydroxy-2-propanon, 1-Hydroxy-2-butanon, 3-Hydroxy-2-butanon und Benzoin.

Polyhydroxyfunktionalisierte Ketone sind erfindungsgemäß bevorzugt die sogenannten Ketosen und werden besonders bevorzugt ausgewählt aus mindestens einem Vertreter aus der Gruppe 1,3-Dihydroxyaceton, D-Psicose, D-Fructose, D-Sorbose, D-Tagatose, D-Ribulose, D-Xylulose, D-Erythrulose sowie den L-Konfigurationen L-Psicose, L-Fructose, L-Sorbose, L-Tagatose, L-Ribulose, L-Xylulose, L-Erythrulose.

Bevorzugte alicyclische, aromatische oder heterocyclische Ketone werden bevorzugt ausgewählt aus mindestens einem Vertreter aus der Gruppe
- Benzylidenketone, insbesondere den Benzylidenketonen aus der Patentanmeldung DE-A1-19717281 (siehe DE-A1-19717281: Formel (I) des 1. Anspruchs und die Vertreter gemäß 4. Anspruch), auf die ausdrücklich Bezug genommen wird,
- Imidazolin-2,4-dion und dessen Derivaten, insbesondere Allantoin und/oder 5,5-Dimethylhydantoin.

Bevorzugte alicyclische, aromatische oder heterocyclische Aldehyde werden bevorzugt ausgewählt aus mindestens einem Vertreter aus der Gruppe
- Benzaldehyd und seinen Derivaten,
- Zimtaldehyd und seinen Derivaten sowie
- Naphthaldehyd und seinen Derivaten.

Die Derivate der Benzaldehyde, Naphthaldehyde bzw. Zimtaldehyde werden bevorzugt ausgewählt aus mindestens einem Vertreter aus der Gruppe Benzaldehyd, Naphthaldehyd, 2-Methylbenzaldehyd, 3-Methylbenzaldehyd, 4-Methylbenzaldehyd, 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methylbenzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholino-benzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 3-Allyl-4-hydroxybenzaldehyd, 3-Allyl-4-hydroxy-5-methoxybenzaldehyd, 3-Allyl-4-hydroxy-5-methylbenzaldehyd, 3-Allyl-5-brom-4-hydroxybenzaldehyd, 3,5-Diallyl-4-hydroxybenzaldehyd, 3-Allyl-4-hydroxy-5-formylbenzaldehyd (5-Allyl-4-hydroxyisophthalaldehyd) und Piperonal.

Alle Aldehyd bzw. Ketoverbindungen sollen aus physiologisch verträglichen bzw. nicht toxischen Verbindungen ausgewählt werden, wenn das erfindungsgemäße Mittel als kosmetisches Mittel Verwendung finden soll.

Die Verbindungen, ausgewählt aus Aldehyden oder Ketonen, sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, in dem erfindungsgemäßen Mittel enthalten.

Als Träger für das erfindungsgemäße Mittel eignen sich bevorzugt flüssige Medien, in denen das erfindungsgemäße Sulfinsäurederivat bevorzugt löslich ist, wie beispielsweise Wasser oder organische Lösemittel. Es ist erfindungsgemäß bevorzugt, wenn der Träger ein kosmetischer Träger ist.

Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.
Ein wässriger kosmetischer Träger enthält mindestens 50 Gew.-% Wasser.
Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Weitere alkoholische Lösemittel, sind beispielsweise Methoxybutanol, Benzylalkohol, 2-Phenoxyethanol, Ethyldiglykol oder 1,2-Propylenglykol. In einer bevorzugten Ausführungsform enthält das erfindungsgemäße Mittel zusätzlich als Lösemittel mindestens einen (C₂ bis C₆)-Alkylmonoalkohol und/oder ein (C₂ bis C₆)-Alkandiol, insbesondere Ethanol, Isopropanol und/oder 1,2-Propylenglykol.

Das erfindungsgemäße Mittel besitzt bevorzugt einen pH-Wert von pH 1 bis pH 9, insbesondere von pH 1,5 bis pH 6. Als pH-Stellmittel eignen sich beispielsweise Genußsäuren, wie Zitronensäure, Weinsäure, Milchsäure oder Äpfelsäure, oder Phosphorsäure, sowie Ammoniak, Alkalimetasilikate, Alkalihydroxide oder Alkanolamine, wie 2-Aminoethanol oder 2-Amino-2-methylpropanol.

In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO(-)- oder -SO₃(-)-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Mitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Bestandteile des wasserhaltigen Trägers werden zur Herstellung der erfindungsgemäßen Mittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Mittels eingesetzt.

Ein zweiter Gegenstand der Erfindung ist die Verwendung des Mittels gemäß erstem Erfindungsgegenstand zur Entfärbung von Substraten, die mit natürlichen und/oder synthetischen Farbstoffen eingefärbt sind.

Das Substrat enthält bevorzugt synthetische Fasern und/oder natürliche Fasern.
Die natürlichen Fasern werden bevorzugt ausgewählt aus cellulosehaltigen Fasern, insbesondere Baumwolle, und keratinhaltigen Fasern, insbesondere Wolle oder tierischen oder menschlichen Haaren, ganz besonders bevorzugt aus menschlichen Haaren.
Die synthetischen Fasern werden bevorzugt ausgewählt werden aus Polyester, Polyamid (wie beispielsweise Nylon), Elastan, Viskose oder Polyacryl.

Die zu entfärbenden Substrate sind bevorzugt mit Oxidationsfarbstoffen und/oder direktziehenden Farbstoffen, als Vertreter der synthetischen Farbstoffe, gefärbt.
In einer weiteren bevorzugten Ausführungsform wurden die zu entfärbenden Substrate mit einem oxidativen Färbemittel gefärbt, welches neben mindestens einer Entwicklerkomponente zusätzlich mindestens eine Kupplerkomponente enthält.
Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlorresorcin, 2-Chlor-6-methyl-3-aminophenol, 2-Amino-3-hydroxypyridin, 2-Methylresorcin, 5-Methylresorcin und 2-Methyl-4-chlor-5-aminophenol.
Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Zusätzlich können die zu entfärbenden Substrate mit Vorstufen naturanaloger Farbstoffe bevorzugt unter Zuhilfenahme solcher Indole und Indoline gefärbt sein, die bevorzugt mindestens zwei Hydroxy- oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.
Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.
Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.
Das zu entfärbende Substrat kann ebenso mit direktziehenden Farbstoffen eingefärbt worden sein. Als direktziehende Farbstoffe kommen dabei insbesondere Nitrophenylendiamine, Nitroaminophenole, Azofarbstoffe, Anthrachinone oder Indophenole in Frage. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahy-drochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.
Ferner können die zu entfärbenden Substrate bevorzugt mit einem kationischen direktziehenden Farbstoff gefärbt sein. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Ein dritter Gegenstand der Erfindung ist ein Verfahren zur reduktiven Entfärbung von mit natürlichen und/oder synthetischen Farbstoffen eingefärbten Substraten, in dem ein Mittel des ersten Erfindungsgegenstandes auf das eingefärbte Substrat aufgetragen wird und nach einer Einwirkzeit wieder abgespült wird.

Die Einwirkzeit beträgt bevorzugt 1 bis 60 Minuten, bevorzugt 5 bis 30 Minuten. Die Einwirkung des erfindungsgemäßen Mittels kann nicht nur bei Raumtemperatur, sondern bevorzugt in einem Temperaturbereich von 15 bis 60 °C, insbesondere von 25 bis 60 °C erfolgen.
Nach Ablauf der Einwirkzeit werden die Haare ausgespült, wobei bevorzugt ein tensidhaltiges Mittel, wie beispielsweise ein Reinigungsmittel oder ein Shampoo, Anwendung findet. Gegebenenfalls kann das Substrat mehrfach ausgespült, bzw. mit dem tensidhaltigen Mittel behandelt werden.
Nach dem Ausspülen kann es vorteilhaft sein, das Substrat mit einer Oxidationsmittelhaltigen Zusammensetzung zu behandeln. Bevorzugt wird Wasserstoffperoxid als Oxidationsmittel, bevorzugt in Konzentrationen von 0,5 bis 6 Gew.-%, eingesetzt. Die Einwirkzeit beträgt bevorzugt 1 bis 30 Minuten, besonders bevorzugt 1 bis 10 Minuten. Nach Ablauf der Einwirkzeit wird die Oxidationsmittelhaltige Zusammensetzug ausgespült.

Als bevorzugte eingefärbte Substrate eignen sich diejenigen, die im zweiten Erfindungsgegenstand definiert wurden.

Als bevorzugte natürliche oder synthetische Farbstoffe wurden bevorzugt diejenigen Farbstoffe verwendet, wir sie im zweiten Erfindungsgegenstand definiert wurden.

Der Erfindungsgegenstand soll exemplarisch anhand der folgenden Ausführungen erläutert werden.

Es wurden die nicht erfindungsgemäßen Entfärbemittel gemäß Tabelle 1 bereitgestellt. Dabei wurden unter anderem folgende Handelsprodukte verwendet:

| Handelsprodukt: | Inhalt | Hersteller: |
|---|---|---|
| Aromox^{®} MCDW | Dimethyl(kokosnussölalkyl)aminoxid; 30 Gew.-% Aktivsubstanz in Wasser (INCI-Bezeichnng: Cocamine Oxide) | Akzo Nobel |

Alle Rohstoffe wurden nach und nach in 75 Gew.-% Wasser unter Rühren gemischt. Anschließend wurde der pH-Wert eingestellt und ggf. mit Wasser auf 100 Gew.-% aufgefüllt.

**Tabelle 1: Entfärbemittel**

| Rohstoff | E1 | V1 |
|---|---|---|
| | [Gew.-%] | [Gew.-%] |
| 2-Hydroxy-2-sulfinoessigsäure, Natriumsalz | 5,0 | 5,0 |
| Natriumsulfit | - | 2,2 |
| Aromox^{®} MCDW | 3,0 | 3,0 |
| H₃PO₄ | ad pH 2,7 | ad pH 2,7 |
| Wasser | ad 100 | ad 100 |

Folgende Prozedur wurde für alle Entfärbemittel der Tabelle 1 durchgeführt:
Eine Haarsträhne (Naturhaar, Fa. Kerling) wurde mit einer handelsüblichen oxidativen Haarfarbe (Igora^{®} Royal (Schwarzkopf), Färbecreme Naturton im Gewichtsverhältnis 1 zu 1 mit dem Entwickler Oxigenta^{®} (6 Gew.-% Wasserstoffperoxid, Fa. Schwarzkopf) über eine Einwirkzeit von 30 Minuten bei Raumtemperatur gefärbt, gespült und für 24 Stunden gelagert. Danach wurde die Strähne in 30 mL eines Entfärbemittels der Tabelle 1 über eine Einwirkzeit von 30 Minuten bei Raumtemperatur getaucht, anschließend mit viel Wasser gespült und an der Luft getrocknet.
Alle Haarsträhnen zeigten nach der Entfärbeprozedur eine hervorragende Aufhellung. Die mit dem Entfärbemittel E1 entfärbten Strähnen weisen eine geringere Haarschädigung auf, als die mit dem Entfärbemittel V1 entfärbten Strähnen.

## Patentansprüche

1. Mittel zum reduktiven Farbabzug, enthaltend in einem Träger mindestens ein Sulfinsäurederivat der Formel (la-8) 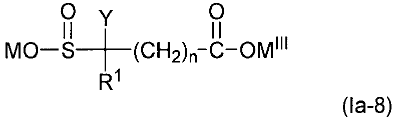 worin
M steht für ein Wasserstoffatom oder ein Äquivalent eines ein- oder mehrwertigen Kations,
Y für eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe steht,
M'" steht unabhängig von M für die unter M aufgeführten Merkmale,
R¹ für ein Wasserstoffatom oder eine Methylgruppe steht,
n für eine ganze Zahl 0, 1, 2, 3, 4, 5 oder 6 steht,
wobei höchstens 1,5 Gew.-% an Sulfitanionen, bezogen auf das Gewicht des anwendungsbereiten Mittels, enthalten sind und
wobei es zusätzlich Verbindungen, ausgewählt aus den Aldehyden und/oder den Ketonen, enthält.

2. Mittel nach Anspruch 1, **dadurch gekennzeichnet, daß** in Formel (la-8) M und M^{III} unabhängig voneinander stehen für ein Wasserstoffatom, ein Ammoniumion, ein Alkalimetallion, für ein halbes Äquivalent eines Erdalkalimetallions oder ein halbes Äquivalent eines Zinkions, insbesondere für ein Wasserstoffatom, ein Ammoniumion, ein Natriumion, ein Kaliumion, ½ Kalziumion, ½ Magnesiumion oder ½ Zinkion.

3. Mittel nach einem der Ansprüche 1 oder 2, **dadurch gekennzeichnet, daß** gemäß Formel (la-8) Y eine Hydroxygruppe oder eine Gruppe -NH₂ bedeutet.

4. Mittel nach einem der Ansprüche 1 bis 3, **dadurch gekennzeichnet, daß** es das Sulfinsäurederivat in einer Menge von 0,01 bis 20 Gew.-%, besonders bevorzugt von 1 bis 20 Gew.-%, jeweils bezogen auf das Gewicht des anwendungsbereiten Mittels, enthält.

5. Mittel nach einem der Ansprüche 1 bis 4, **dadurch gekennzeichnet, daß** es höchstens 1,0 Gew.-%, insbesondere höchstens 0,5 Gew.-%, jeweils bezogen auf das Gewicht des anwendungsbereiten Mittels, an Sulfitionen enthält.

6. Mittel nach einem der Ansprüche 1 bis 5, **dadurch gekennzeichnet, daß** es einen pH-Wert von pH 1 bis pH 9, insbesondere von pH 1,5 bis pH 6, besitzt.

7. Verwendung eines Mittels gemäß einem der Ansprüche 1 bis 6 zur Entfärbung von Substraten, die mit natürlichen und/oder synthetischen Farbstoffen eingefärbt sind.

8. Verwendung nach Anspruch 7, **dadurch gekennzeichnet, daß** die zu entfärbenden Substrate mit Oxidationsfarbstoffen und/oder direktziehenden Farbstoffen gefärbt sind.

9. Verfahren zur reduktiven Entfärbung von mit natürlichen und/oder synthetischen Farbstoffen eingefärbten Substraten, **dadurch gekennzeichnet, daß** ein Mittel gemäß einem der Ansprüche 1 bis 6 auf das eingefärbte Substrat aufgetragen wird und nach einer Einwirkzeit wieder abgespült wird.

10. Verfahren nach Anspruch 9, **dadurch gekennzeichnet, daß** die zu entfärbenden Substrate mit Oxidationsfarbstoffen und/oder direktziehenden Farbstoffen eingefärbt sind.

## Claims

1. An agent for reductive color removal, said agent containing, in a carrier, at least one sulfinic acid derivative of formula (la-8) 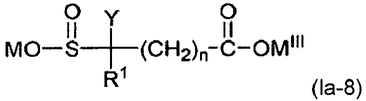 in which
M represents a hydrogen atom or an equivalent of a monovalent or polyvalent cation,
Y represents a hydroxy group, an -NH₂ group or a group -NR³R⁴, wherein R³ and R⁴, independently of one another, represent a (C₁ to C₆) alkyl group, a (C₂ to C₆) alkenyl group, a (C₁ to C₆) hydroxy alkyl group, a (C₂ to C₆) polyhydroxy alkyl group, an aryl group or an aryl (C₁ to C₆) alkyl group.
M'" independently of M, represents the features listed under M,
R¹ represents a hydrogen atom or a methyl group,
n represents an integer 0, 1, 2, 3, 4, 5 or 6,
wherein no more than 1.5 wt.% sulfite anions, based on the weight of the ready-to-use agent, are contained and
wherein the agent additionally contains compounds selected from aldehydes and/or ketones.

2. The agent according to claim 1, **characterized in that**, in formula (la-8), M and M'", independently of one another, represent a hydrogen atom, an ammonium ion, an alkali metal ion, a half-equivalent of an alkaline earth metal ion or a half-equivalent of a zinc ion, in particular a hydrogen atom, an ammonium ion, a sodium ion, a potassium ion, ½ a calcium ion, ½ a magnesium ion, or ½ a zinc ion.

3. The agent according to one of claims 1 or 2, **characterized in that**, according to formula (la-8), Y represents a hydroxy group or an -NH₂ group.

4. The agent according to one of claims 1 to 3, **characterized in that** said agent contains the sulfinic acid derivative in an amount of from 0.01 to 20 wt.%, more preferably from 1 to 20 wt.%, in each case based on the weight of the ready-to-use agent.

5. The agent according to one of claims 1 to 4, **characterized in that** it contains no more than 1.0 wt.%, in particular no more than 0.5 wt.% sulfite ions, in each case based on the weight of the ready-to-use agent.

6. The agent according to one of claims 1 to 5, **characterized in that** it has a pH value of from pH 1 to pH 9, in particular from pH 1.5 to pH 6.

7. The use of an agent according to one of claims 1 to 6 for removing color from substrates that are dyed using natural and/or synthetic dyes.

8. The use according to claim 7, **characterized in that** the substrates from which color is to be removed are colored using oxidation dyes and/or substantive dyes.

9. A method for reductive color removal from substrates dyed using natural and/or synthetic dyes, **characterized in that** an agent according to one of claims 1 to 6 is applied to the dyed substrate and is rinsed off again after an exposure time.

10. The method according to claim 9, **characterized in that** the substrates from which color is to be removed are dyed using oxidation dyes and/or substantive dyes.

## Revendications

1. Agent de décoloration par réduction, contenant dans un support au moins un dérivé d'acide sulfinique de formule (la-8) 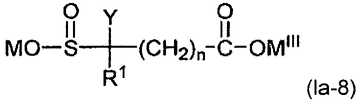 dans laquelle
M représente un atome d'hydrogène ou un équivalent d'un cation monovalent ou polyvalent,
Y représente un groupe hydroxy, un groupe -NH₂ ou un groupe -NR³R⁴, R³ et R⁴ représentant indépendamment l'un de l'autre un groupe alkyle (en C₁ à C₆), un groupe alcényle (en C₂ à C₆), un groupe hydroxyalkyle (en C₁ à C₆), un groupe polyhydroxyalkyle (en C₂ à C₆), un groupe aryle ou un aryl-alkyle (en C₁ à C₆),
M'" représente, indépendamment de M, les caractéristiques énoncées pour M,
R¹ représente un atome d'hydrogène ou un groupe méthyle,
n représente le nombre entier 0, 1, 2, 3, 4, 5 ou 6,
1,5 % en poids d'anions sulfites maximum étant présents, par rapport au poids de l'agent prêt à l'emploi, et
l'agent contenant en plus des composés sélectionnés parmi les aldéhydes et/ou les cétones.

2. Agent selon la revendication 1 **caractérisé en ce que**, dans la formule (la-8), M et M^{III} représentent, indépendamment l'un de l'autre, un atome d'hydrogène, un ion ammonium, un ion de métal alcalin, la moitié d'un équivalent d'un ion de métal alcalino-terreux ou la moitié d'un équivalent d'un ion zinc, en particulier un atome d'hydrogène, un ion ammonium, un ion sodium, un ion potassium, ½ ion calcium, ½ ion magnésium, ou ½ ion zinc.

3. Agent selon l'une quelconque des revendications 1 ou 2 **caractérisé en ce que** selon la formule (la-8), Y représente un groupe hydroxy ou un groupe -NH₂.

4. Agent selon l'une quelconque des revendications 1 à 3 **caractérisé en ce qu'**il contient le dérivé d'acide sulfinique dans une proportion de 0,01 à 20 % en poids, de manière particulièrement préférée de 1 à 20 % en poids, par rapport, respectivement, au poids de l'agent prêt à l'emploi.

5. Agent selon l'une quelconque des revendications 1 à 4 **caractérisé en ce qu'**il contient maximum 1,0 % en poids, en particulier maximum 0,5 % en poids d'ions sulfite, par rapport, respectivement, au poids de l'agent prêt à l'emploi.

6. Agent selon l'une quelconque des revendications 1 à 5 **caractérisé en ce qu'**il possède un pH de 1 à 9, en particulier un pH de 1,5 à 6.

7. Utilisation d'un agent selon l'une quelconque des revendications 1 à 6 pour la décoloration de substrats qui sont teints avec des colorants naturels et/ou synthétiques.

8. Utilisation d'un agent selon la revendication 7 **caractérisée en ce que** les substrats à décolorer sont teints avec des colorants d'oxydation et/ou des colorants directs.

9. Procédé de décoloration par réduction de substrats teints avec des colorants naturels et/ou synthétiques **caractérisé en ce qu'**un agent selon l'une quelconque des revendications 1 à 6 est appliqué sur le substrat teint et qu'il est rincé après une certaine durée d'action.

10. Procédé selon la revendication 9 **caractérisé en ce que** les substrats à décolorer sont teints avec des colorants d'oxydation et/ou des colorants directs.
